# EUROPEAN PATENT SPECIFICATION

(11) **EP 0 858 781 B1**
(45) Date of publication and mention of the grant of the patent: **09.01.2002**
(21) Application number: 97122922.4
(22) Date of filing: 28.12.1997
(51) Int. Cl.: A61B 17/60, A61B 17/66

(54) **Orthopaedic device for the gradual correction of limbs**
Orthopädische Vorrichtung zur allmählichen Korrektur von Gliedern
Appareil orthopedique pour la correction graduelle des membres

(30) Priority: 14.02.1997 IT VR970013
(43) Date of publication of application: 19.08.1998
(73) Proprietor: ORTHOFIX S.r.l., 37012 Bussolengo (VR) (IT)
(72) Inventor: Faccioli, Giovanni, 46040, Monzambano, Mantova (IT); Venturini, Daniele, 37065 Povegliano Veronese, Verona (IT); Ten Veldhuis, Sander, 37138 Verona (IT)
(74) Representative: Botti, Mario

(56) References cited:
- EP-A- 0 385 929
- EP-A- 0 699 419
- WO-A-92/02184
- FR-A- 2 645 428
- US-A- 5 152 280

## Description

### Technical Field

The present invention relates to an orthopaedic device for the gradual correction of limbs, in accordance with the preamble of the claim 1.

### Background Art

It is known that external fixation devices, adjustable in length and angular attitude, are commonly utilised for correcting certain angular and longitudinal deformations of long bones of limbs. Such fixation devices essentially comprise clamps which hold groups of bone screws inserted in the portions of the bone affected by defects, such clamps being slidably mounted on elements or guides longitudinally positionable externally to the limb to be treated.

The correction is normally carried out gradually with the aid of compressor-distractor devices which act on the mobile clamps while the bone callous regenerates itself permitting its manipulation until the desired correction is obtained.

One known device for the gradual correction of the limbs has a lateral clamp which allows an angular correction of the bone. Such known device has the drawback that the angular rotation induces a shifting of the screws in a transverse direction which renders the correction of the limb difficult.

Orthopaedic devices according to the preamble of Claim 1 are shown in EP-A-0 699 419 or EP-A-0 385 929.

### Disclosure of the Invention

One principal aim of the present invention is to overcome the above described drawbacks providing an orthopaedic device which allows a complete correction in geometric planes which are also inclined with respect to the lateral and front-rear planes, in a gradual and easily controllable manner.

With this principal aim and others in view, there is provided an orthopaedic device for the gradual correction of angular and longitudinal defects of elongated bones, comprising at least one first clamp for a first group of screws insertable in a proximal portion of a bone, and at least one second clamp for a second group of screws insertable in a distal portion of the bone, a longitudinal guide bar positioned externally of the limb to be corrected for slidably supporting said first and second clamps in longitudinally distanced positions, at least one of said clamps being selectively orientatable about a substantially transverse axis for carrying out angular corrections of the bone, there being provided compression/distraction means movably coupleable with said clamps for carrying out longitudinal corrections of the bone, characterised by the fact that said at least one orientatable clamp has angular adjustment means in a predetermined geometric plane, as well as transverse adjustment means for the group of bone screws carried by said clamp parallel to themselves for compensating the lateral movement induced by the angular correction.

### Brief Description of Drawings

The characteristics and advantages of the invention will become apparent from the following description of some preferred but not exclusive embodiments of an intramedullary cavity nail according to the invention, illustrated for illustrative and non-limiting purposes with the help of the attached drawing sheets in which:
Fig. 1 illustrates a general front elevation view of the orthopaedic device according to a preferred aspect of the invention applied to a femur in a lateral position;

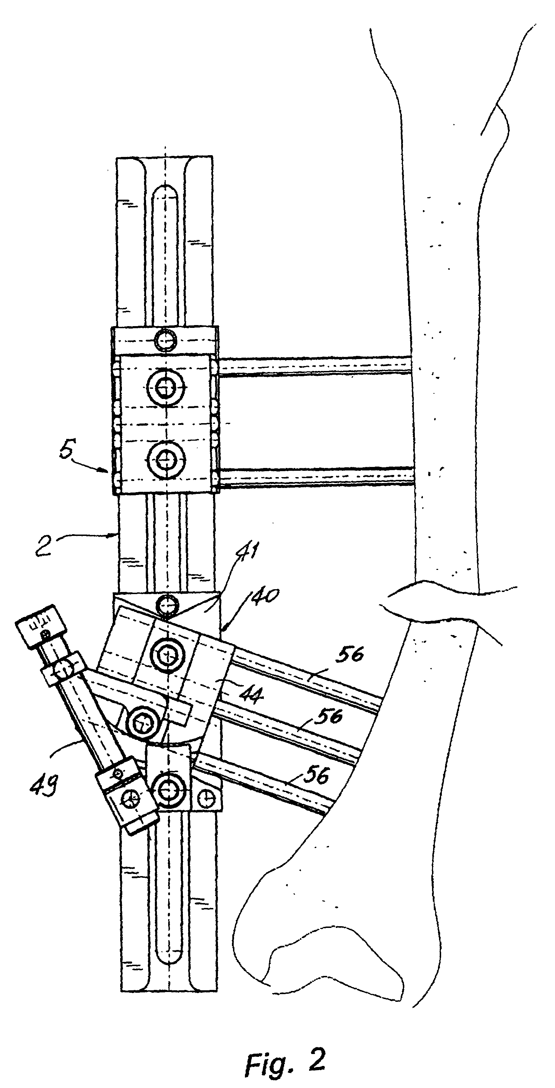

Fig. 2 illustrates a variation of the device of Fig. 1 in enlarged scale and in a correction phase of an angular defect of a femur;

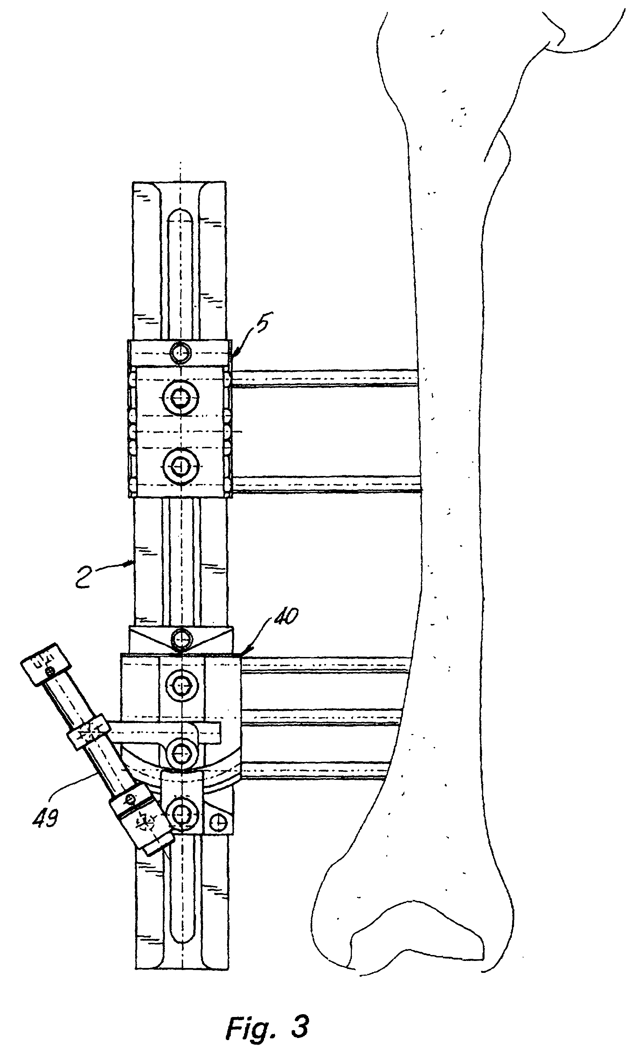

Fig. 3 illustrates the device of Fig. 2 at the end of the correction;
Fig. 4 illustrates the device of Fig. 1 used in a front-rear plane;

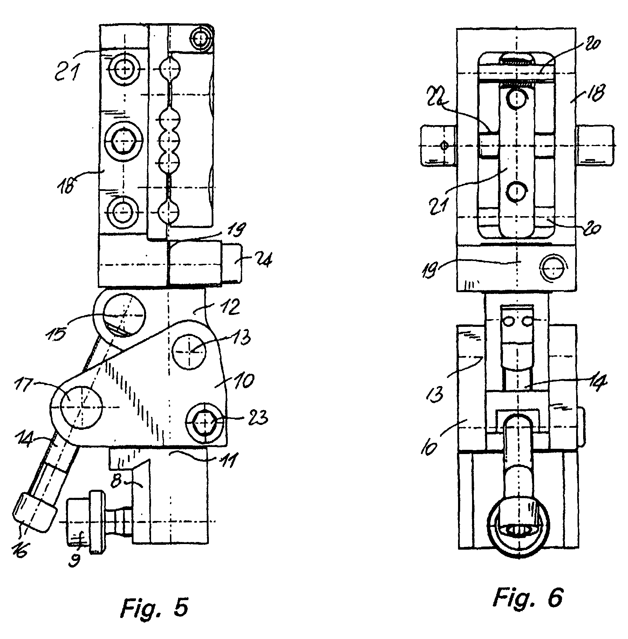

Fig. 5 illustrates an enlarged front elevation view of a clamp of the assembly of Fig. 1;
Fig. 6 illustrates a lateral elevation view of the detail of Fig. 5;

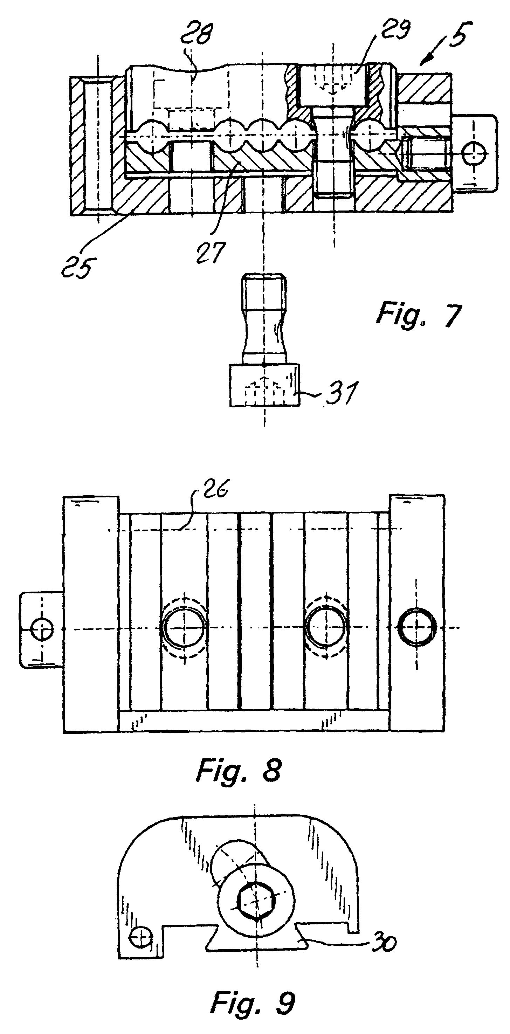

Fig. 7 illustrates a section view of a first variation of the clamp of the assembly of Fig. 1;
Fig. 8 illustrates a top plan view of the detail of Fig. 7;
Fig. 9 illustrates a lateral elevation view of the detail of Fig. 7;
Fig. 10 illustrates a section view of a second variation of the clamp of the assembly of Fig. 1;
Fig. 11 illustrates a top plan view of the detail of Fig. 10;
Fig. 12 illustrates a lateral elevation view of the detail of Fig. 10;

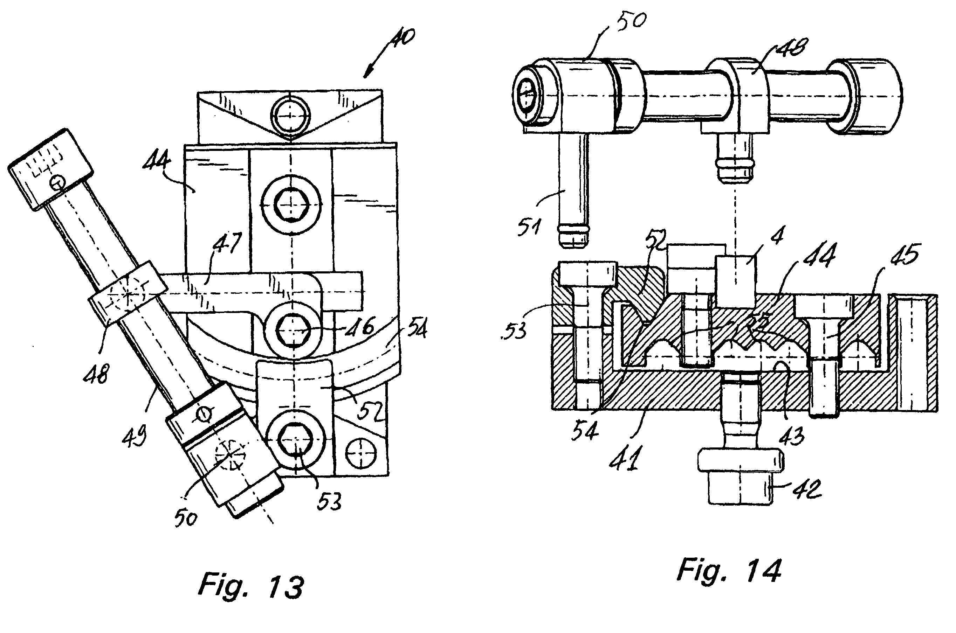

Figs. 13 and 14 illustrate respectively a top plan view and a partially sectional view of a clamp of the device of Figs. 2 and 3.

### Best Modes for Carrying Out the Invention

With reference to the cited figures, an orthopaedic device according to a preferred aspect of the invention, globally indicated by the reference numeral 1, for the gradual correction of angular and longitudinal defects of elongated bones, for example a femur F, essentially comprises a longitudinal guide bar 2 of generally semicircular cross section positioned externally to the limb to be corrected, upon which a first clamp 3 for a first group of screws 4 insertable in a proximal portion of the bone and a second clamp 5 for a second group of screws 6 insertable in a distal portion of the bone are mounted.

The proximal clamp 3 comprises an attachment portion 7 movably anchorable to the bar 2, for example by means of a dovetail tenon 8 coupleable to a longitudinal cavity of the bar 2 and a blocking screw 9, upon which is mounted a bracket 10 rotatable about a first substantially longitudinal axis 11.

An intermediate body 12 is hinged to the bracket 10 by means of a substantially transversal axis 13 for correcting the angular defects, and is micrometrically adjustable by means of a screw 14 having an end 15 hinged to the body 12, the opposite end 16 being provided with a hexagonal fitting and the central portion being engaged in a pivot member 17 united to the bracket 10.

A substantially L-shaped support 18 is rotatably mounted on the intermediate body 12 about a substantially longitudinal second axis 19.

The support 18 has two transverse spines 20 which guide a jaw 21 of the clamp and a micrometric screw adjustment mechanism 22 for promoting the controlled translation of the jaw 21 so as to compensate the lateral movements induced by the angular corrections. Fixed on the jaw 21 by means of screws is a jaw 21' of a per se known type.

Expediently, there are provided stop screws 23, 24 for blocking the respective axes 11 and 19 so as to define a predetermined geometric plane for the angular correction.

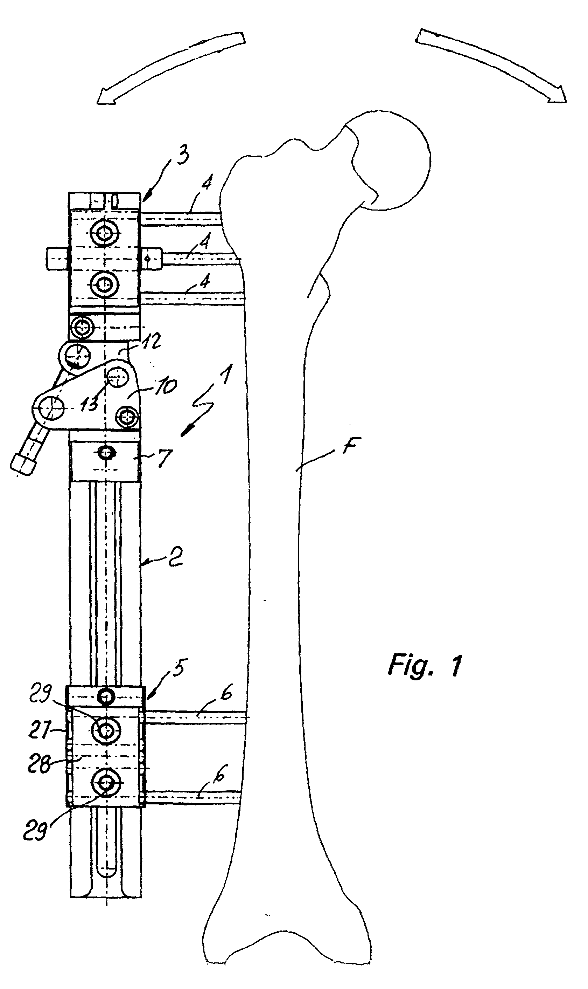

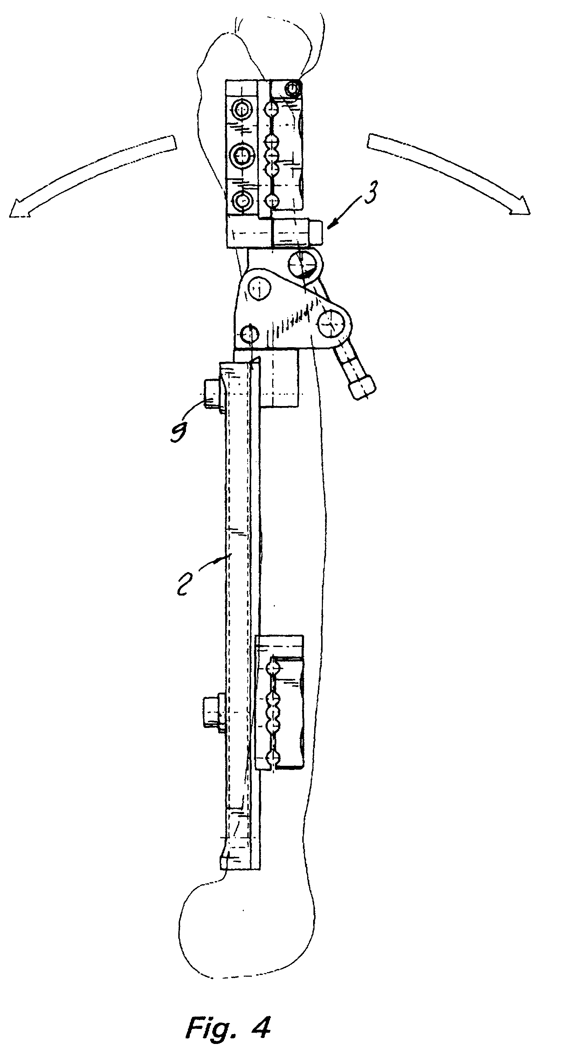

The device of Fig. 1 is shown in Fig. 4 but active in a front-rear plane.

The second clamp 5, which is normally positioned in a distal or medial position, is of the swivelling type and is formed by a support plate 25 anchorable to the bar 2, upon which is hinged about a substantially longitudinal axis 26 a first jaw 27 of the clamp. A second jaw 28 is locked on the first jaw 26 by means of locking screws 29. Both the jaws have transverse seats of a per se known shape for accommodating the screws 6.

The support plate 25 has means for connection to the bar which may be constituted by a dovetail tenon 30 engageable, before the mounting of the other clamp, in a countershaped cavity formed along the bar, and by a stop screw 31 passing through a longitudinal groove of the bar.

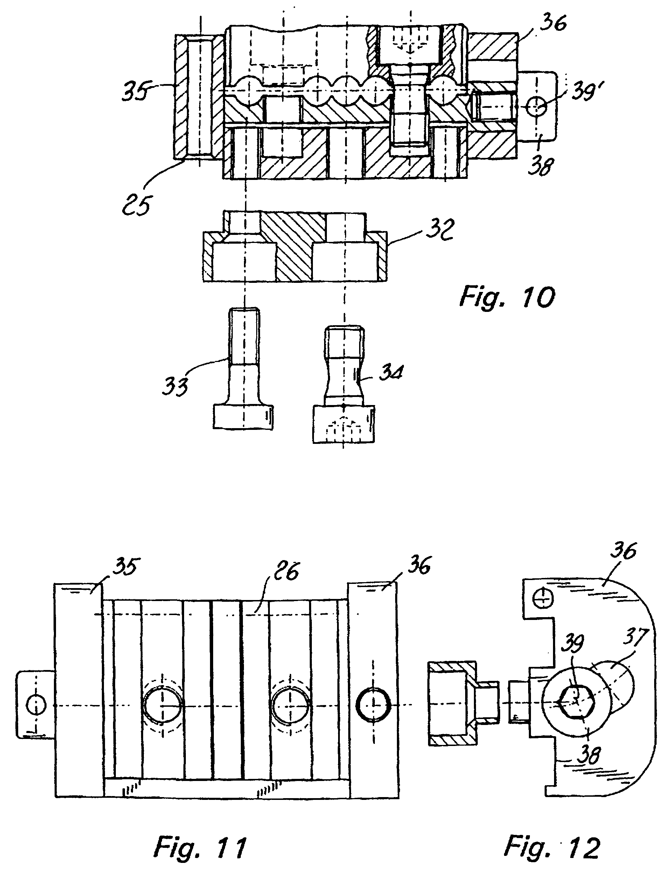

In the alternative embodiment illustrated in the Figures 10 to 12, the connection means of the plate 25 to the bar 2 are constituted by a connecting block 32 anchored to the bar 2 by means of screws 33, 34 of which one serves to anchor the block allowing it to slide along the groove, while the other serves to block the longitudinal movement of the clamp.

The plate has two end supports 35, 36 one of which has an arcuate eyelet hole 37 in which there passes a screw 38 with a hexagonal fitting 39 and also with radial holes 39' for its screwing in case of limited space for the surgeon.

Thanks to its possibility of being able to swivel, the clamp 5 may be inclined allowing for the screws to be screwed into the bone also in case of notable curvature of the same, without having to move the clamp excessively towards the end of the bar.

Another clamp, referenced by 40, is of an oscillating type, and is constituted by a base 41 anchorable to the bar 2 by means of a screw 42 and having a substantially flat external surface 43.

A cover 44 is oscillatingly mounted on the flat base 41 about an axis 45 substantially perpendicular to the longitudinal axis of the bar.

To the cover 44 there is fixed by means of a screw 46 a bracket 47 to whose end is hinged an internally threaded ring 48 in which is engaged a screw 49 having an end held axially by a sleeve 50 with a pin insertable in a hole provided in the base 25. The screw 49 has both ends with hexagonal set heads in order to allow to control it from both sides.

For blocking the base 41 in the pre-established angular position, there is provided a stop 52 anchored to the base 41 by means of a screw 53 and having a cuneiform end which engages against the bottom of a circular cavity 54 with a bottom substantially countershaped with respect to the stop 52, formed on the external face of said cover. In such a manner it is possible to exert notable blocking forces rendering the orientation of the clamp more reliable and secure.

The cover 44 has on its internal face which faces the base 41 five transverse seats 55 for the bone screws, with transverse cross section of greater value than the maximum diameter of the screws and with a V-shaped bottom for permitting the sliding of the screws 56 parallel to themselves in case of loosening of the cover 44.

Obviously, all of the clamps have holes for the insertion of controlling ends of compressors/distractors in order to carry out an elongation of the bones.

## Claims

1. Orthopaedic device for the gradual correction of angular and longitudinal defects of elongated bones, comprising at least one first clamp (3) for a first group of screws insertable in a proximal portion of a bone, and at least one second clamp (5; 40) for a second group of screws insertable in a distal portion of the bone, a longitudinal guide bar (2) positioned externally of the limb to be corrected for slidably supporting said clamps (3; 5; 40) in longitudinally distanced positions, at least one of said clamps being selectively orientatable about a substantially transverse axis for carrying out angular corrections of the bone, there being provided compression/distraction means movably coupleable with said clamps for carrying out longitudinal corrections of the bone, **characterised by** the fact that said at least one orientatable clamp (3) has angular adjustment means (14) in a predetermined geometric plane, as well as transverse adjustment means (22) for the group of bone screws (4) carried by said clamp parallel to themselves for compensating the lateral movement induced by the angular correction.

2. Orthopaedic device according to claim 1, wherein said angular (14) and transverse (22) adjustment means are of a micrometric type.

3. Orthopaedic device according to claim 1, wherein said at least one orientatable clamp (3) has axial orientation means (11; 19) for selectively defining said angular adjustable geometric plane.

4. Orthopaedic device according to claim 1, wherein said at least one orientatable clamp (3) comprises an attachment portion (8) movably anchorable to said longitudinal bar (2) upon which there is mounted a bracket (10) pivotable about a substantially longitudinal first axis (11).

5. Orthopaedic device according to claim 1, wherein on said bracket (10) there is hinged about a substantially transverse axis (13) an intermediate body (12) united to said bracket by means of a screw and female thread micrometric adjusting mechanism (14).

6. Orthopaedic device according to claim 1, wherein on said intermediate body (12) there is mounted a substantially L-shaped support (18) rotatable about a substantially longitudinal second axis (19).

7. Orthopaedic device according to claim 1, wherein said support (18) has transverse guiding means (20) for a jaw (21) of said clamp and a screw and female thread micrometric adjustment mechanism (22) for promoting the controlled translation of said jaw (21) along said transverse guiding means.

8. Orthopaedic device according to claim 1, wherein there are provided stop means (23, 24) for each of said substantially longitudinal rotation axes for selectively determining the angular adjustment plane of the clamp.

9. Orthopaedic device according to claim 1, wherein the other one of said clamps (40) is of the oscillating type and has a base (41) anchorable to said bar (2) with a substantially flat external surface (43).

10. Orthopaedic device according to claim 1, wherein on said base (41) there is mounted a cover (44) oscillable about an axis (45) substantially perpendicular to the longitudinal axis of said bar.

11. Orthopaedic device according to claim 1, wherein there are provided screw and female thread adjustment means (49) adapted to selectively control the angular position of said cover (44) with respect to said base (41), with said screw (49) provided with holding means towards both its longitudinal ends.

12. Orthopaedic device according to claim 1, wherein there is provided a cuneiform stop (52) screwable to said base for selectively engaging on the bottom of a circular seat (54) with a bottom substantially countershaped with respect to the stop formed on the external face of said cover (44).

13. Orthopaedic device according to claim 1, wherein said cover has on the internal face which faces said base a plurality of transverse seats (55) for the bone screws, with transverse sections of greater size than the maximum diameter of the screws and with a V-shaped bottom for allowing the screws to slide parallel to themselves in case of loosening of the cover.

14. Orthopaedic device according to claim 1, wherein there is provided a further clamp (5) for locking a group of screws (6) insertable in medial and distal portions of the bone.

15. Orthopaedic device according to claim 14, wherein said medial clamp (5) is of the basculant type, and is formed by a support plate (25) anchorable to said bar to which is hinged with a substantially longitudinal axis (26) a first jaw (27) of said clamp to which is coupleable a second jaw (28) by means of locking screws (29).

16. Orthopaedic device according to claim 15, wherein said support plate (25) has means for connecting to said bar constituted by a dovetail tenon (30) engageable, before the mounting of the other clamps, in a countershaped seat formed along said bar and by a stop screw (31) passing through a longitudinal groove of said bar.

17. Orthopaedic device according to claim 15, wherein said support plate (25) has means for connecting to said bar (2) constituted by a connection block (32) screwed to said plate for blocking the separation of the bar (2) also after the mounting of the other clamps.

## Patentansprüche

1. Orthopädische Vorrichtung für die allmähliche Korrektur von Winkel- und Längsfehlern von langgestreckten Knochen, die mindestens eine erste Klemme (3) für eine erste Gruppe von Schrauben, die in einen nächstgelegenen Abschnitt eines Knochens eingesetzt werden können, und mindestens eine zweite Klemme (5; 40) für eine zweite Gruppe von Schrauben, die in einen entfernten Abschnitt des Knochens eingesetzt werden können, und eine längliche Führungsstange (2), die sich außerhalb des Körperglieds befindet, das korrigiert werden soll, zum gleitbaren Tragen der Klemmen (3; 5; 40) in längsseits voneinander beabstandeten Positionen aufweist, wobei zumindest eine der Klemmen selektiv ausrichtbar um eine im wesentlichen querliegende Achse zum Ausführen von Winkelkorrekturen des Knochens ist, wobei eine Druck-/Trenn-Einrichtung vorgesehen ist, die beweglich koppelbar mit den Klemmen zum Ausführen von Längskorrekturen des Knochens ist, **gekennzeichnet durch** das Merkmal, dass die mindestens eine ausrichtbare Klemme (3) eine Winkeleinstelleinrichtung (14) in einer vorgegebenen, geometrischen Ebene und auch eine Quereinstelleinrichtung (22) für die Gruppe von Knochenschrauben (4), die von der Klemme parallel zueinander getragen werden, zum Kompensieren der lateralen Bewegung hat, die **durch** die Winkelkorrektur erzeugt wird.

2. Orthopädische Vorrichtung nach Anspruch 1, worin die Winkeleinstelleinrichtung (14) und die Quereinstelleinrichtung (22) vom Mikrometer-Typ sind.

3. Orthopädische Vorrichtung nach Anspruch 1, worin die mindestens eine ausrichtbare Klemme (3) eine axiale Ausrichteinrichtung (11; 19) zum selektiven Festlegen der winkelmäßig einstellbaren geometrischen Ebene hat.

4. Orthopädische Vorrichtung nach Anspruch 1, worin die mindestens eine ausrichtbare Klemme (3) einen Anbringungsabschnitt (8) aufweist, der an der Längsstange (2) beweglich befestigbar ist und an dem ein Träger (10) angebracht ist, der um eine im wesentlichen längsgerichtete, erste Achse (11) drehbar ist.

5. Orthopädische Vorrichtung nach Anspruch 1, worin an dem Träger (10) ein Zwischenkörper (12) gelenkig um eine im wesentlichen quergerichtete Achse (13) angebracht ist, der mit dem Träger mittels einer Schraube und der mikrometrischen Einstelleinrichtung (14) mit Gegengewinde verbunden ist.

6. Orthopädische Vorrichtung nach Anspruch 1, worin an dem Zwischenkörper (12) ein im wesentlichen L-förmiger Träger (18) angebracht ist, der um eine im wesentlichen längsgerichtete, zweite Achse (19) drehbar ist.

7. Orthopädische Vorrichtung nach Anspruch 1, worin der Träger (18) eine quergerichtete Führungseinrichtung (20) für eine Klemmbacke (21) der Klemme und eine Schraube und eine mikrometrische Einstelleinrichtung (22) mit Gegengewinde zum Unterstützen der gesteuerten Translation der Klemmbacke (21) entlang der quergerichteten Führungseinrichtung hät.

8. Orthopädische Vorrichtung nach Anspruch 1, worin Anschlagmittel (23, 24) für jede der im wesentlichen längsgerichteten Drehachsen zum selektiven Bestimmen der Winkeleinstellungsebene der Klemme vorgesehen sind.

9. Orthopädische Vorrichtung nach Anspruch 1, worin die andere der Klemmen (40) vom beweglichen Typ ist und eine Basis (41) hat, die an der Stange (2) mit einer im wesentlichen flachen Außenoberfläche (43) befestigbar ist.

10. Orthopädische Vorrichtung nach Anspruch 1, worin eine Abdeckung (44) an der Basis (41) angebracht ist, die um eine Achse (45) beweglich ist, die im wesentlichen rechtwinklig zur Längsachse der Stange ist.

11. Orthopädische Vorrichtung nach Anspruch 1, worin eine Schraube und eine Einstelleinrichtung (49) mit Gegengewinde vorgesehen sind, die für die selektive Steuerung der Winkelposition der Abdeckung (44) bezüglich der Basis (41) ausgelegt sind, wobei die Schraube (49) mit einer Halteeinrichtung zu ihren beiden Längsenden hin versehen ist.

12. Orthopädische Vorrichtung nach Anspruch 1, worin ein keilförmiger Anschlag (52) vorgesehen ist, der in die Basis für einen selektiven Eingriff in einen Boden eines kreisförmigen Sitzes (54) einschraubbar ist, wobei der Boden im wesentlichen die Gegenform mit Bezug auf den Anschlag hat, der an der Außenfläche der Abdeckung (44) ausgebildet ist.

13. Orthopädische Vorrichtung nach Anspruch 1, worin die Abdeckung an der Innenfläche, die der Basis gegenüberliegt, eine Vielzahl von quergerichteten Sitzen (55) für die Knochenschrauben mit quergerichteten Abschnitten größerer Abmessung als dem maximalen Durchmesser der Schrauben und mit einem V-förmigen Boden zum Ermöglichen hat, dass die Schrauben parallel zueinander im Fall eines Lockerns der Abdeckung gleiten können.

14. Orthopädische Vorrichtung nach Anspruch 1, worin eine weitere Klemme (5) zum Sperren bzw. Verblocken einer Gruppe von Schrauben (6) vorgesehen ist, die in mittlere und entfernte Abschnitte des Knochens einsetzbar sind.

15. Orthopädische Vorrichtung nach Anspruch 14, worin die mittlere Klemme (5) vom Baskültyp ist und durch eine Trägerplatte (25), die an der Stange befestigbar ist und an der mit im wesentlichen einer Längsachse (26) eine erste Klemmbacke (27) der Klemme gelenkig angebracht ist, mit der eine zweite Klemmbacke (28) mittels Sperrschrauben (29) koppelbar ist.

16. Orthopädische Vorrichtung nach Anspruch 15, worin die Trägerplatte (25) eine Einrichtung zum Verbinden mit der Stange hat, die durch einen Schwalbenschwanz-Zapfen (30), der vor dem Anbringen der anderen Klemmen in einen Gegensitz eingreift, der entlang der Stange ausgebildet ist, und durch eine Anschlagschraube (31) ausgebildet ist, die durch eine Längsrille der Stange hindurchgeht.

17. Orthopädische Vorrichtung nach Anspruch 15, worin die Trägerplatte (25) eine Einrichtung zum Verbinden mit der Stange (2) hat, die durch einen Verbindungsblock (32) ausgebildet ist, der an der Platte zum Blockieren einer Trennung des Stabes (2) auch nach dem Anbringen der anderen Klemmen verschraubt ist.

## Revendications

1. Dispositif orthopédique pour correction graduelle des défauts angulaires et longitudinaux des os allongés, comprenant au moins un premier clamp (3) pour un premier groupe de vis insérables dans une partie proximale d'un os, et au moins un deuxième clamp (5 ; 40) pour un deuxième groupe de vis insérables dans une partie distale de l'os, une glissière longitudinale (2) positionnée à l'extérieur du membre à corriger, pour supporter longitudinalement lesdits clamps (3 ; 5 ; 40) de manière coulissante et les maintenir dans des positions distantes, au moins un desdits clamps étant orientable sélectivement selon un axe principalement transversal aux fins d'apporter les corrections angulaires de l'os, un moyen de compression/distraction étant prévu pour être couplé en mouvement avec lesdits clamps pour effectuer les corrections longitudinales de l'os, **caractérisé en ce qu'**au moins un clamp orientable (3) est muni d'un moyen de réglage angulaire (14) dans un plan géométrique prédéterminé et d'un moyen de réglage transversal (22) pour le groupe de vis à os (4) porté par ledit clamp, parallèles entre eux, pour compenser le mouvement latéral induit par la correction angulaire.

2. Dispositif orthopédique selon la revendication 1, **caractérisé en ce que** lesdits moyens de réglage angulaire (14) et transversal (22) sont de type micrométrique.

3. Dispositif orthopédique selon la revendication 1, **caractérisé en ce qu'**au moins un clamp orientable (3) est muni d'un moyen d'orientation axiale (11 ; 19) pour définir sélectivement ledit plan géométrique angulaire réglable.

4. Dispositif orthopédique selon la revendication 1, **caractérisé en ce qu'**au moins un clamp orientable (3) comprend une partie de fixation (8) ancrable de façon mobile dans ladite glissière longitudinale (2) sur laquelle une patte d'attache (10) est montée pivotable autour d'un premier axe (11) principalement longitudinal.

5. Dispositif orthopédique selon la revendication 1, **caractérisé en ce que**, sur ladite patte d'attache (10), un corps intermédiaire (12) est articulé selon un axe principalement transversal (13) raccordé à ladite patte d'attache au moyen d'un mécanisme de réglage micrométrique (14) à vis et taraudage.

6. Dispositif orthopédique selon la revendication 1, **caractérisé en ce qu'**un support principalement en forme de L (18) est monté tournant autour d'un second axe principalement longitudinal (19) sur ledit corps intermédiaire (12).

7. Dispositif orthopédique selon la revendication 1, **caractérisé en ce que** ledit support (18) est muni d'un moyen de guidage transversal (20) pour la mâchoire de serrage (21) dudit clamp et d'un mécanisme (22) de réglage micrométrique à vis et taraudage pour assurer la translation contrôlée de ladite mâchoire (21) le long dudit moyen de guidage transversal.

8. Dispositif orthopédique selon la revendication 1, **caractérisé en ce qu'**un moyen d'arrêt (23, 24) est prévu pour chacun desdits axes de rotation principalement longitudinaux afin de déterminer sélectivement le plan de réglage angulaire du clamp.

9. Dispositif orthopédique selon la revendication 1, **caractérisé en ce que** l'autre clamp (40) est de type oscillant et présente une base (41) ancrable dans ladite glissière (2) avec une surface externe principalement plate (43).

10. Dispositif orthopédique selon la revendication 1, **caractérisé en ce qu'**un couvercle d'obturation (44) est monté sur ladite base (41) oscillant selon un axe (45) principalement perpendiculaire à l'axe longitudinal de ladite glissière.

11. Dispositif orthopédique selon la revendication 1, **caractérisé en ce qu'**un moyen de réglage à vis et taraudage (49) adapté est prévu pour contrôler sélectivement la position angulaire dudit couvercle (44) par rapport à ladite base (41), lesdites vis (49) étant munies d'un moyen de blocage vers leurs deux extrémités longitudinales.

12. Dispositif orthopédique selon la revendication 1, **caractérisé en ce qu'**un arrêt cunéiforme (52) est prévu vissable à ladite base pour s'engager sélectivement sur le fond d'un siège circulaire (54) dont le fond usiné sensiblement de manière complémentaire par rapport au butoir formé sur la face externe dudit couvercle (44).

13. Dispositif orthopédique selon la revendication 1, **caractérisé en ce que** ledit couvercle présente, sur la face interne qui est adjacente à ladite base, plusieurs sièges transversaux (55) pour les vis à os, avec des sections transversales de plus grande dimension que le diamètre maximum des vis et avec un fond en forme de V pour permettre aux vis de glisser parallèlement à elles-mêmes en cas de desserrement du couvercle.

14. Dispositif orthopédique selon la revendication 1, **caractérisé en ce qu'**un autre clamp (5) est prévu pour verrouiller un groupe de vis (6) insérables dans les parties médiales et distales de l'os.

15. Dispositif orthopédique selon la revendication 14, **caractérisé en ce que** ledit clamp médial (5) est de type basculant et est formé par une plaque support (25) ancrable dans ladite glissière sur laquelle est articulée, selon un axe principalement longitudinal (26), une première mâchoire de serrage (27) dudit clamp auquel une seconde mâchoire (28) peut être couplée au moyen d'une vis de blocage (29).

16. Dispositif orthopédique selon la revendication 15, **caractérisé en ce que** ladite plaque d'appui (25) comporte un moyen de raccordement à ladite glissière constitué par un tenon en queue d'aronde (30) engageable, avant le montage des autres clamps, dans un siège usiné de manière complémentaire, formé le long de ladite glissière et par une vis de blocage (31) passant par une rainure longitudinale de ladite glissière.

17. Dispositif orthopédique selon la revendication 15, **caractérisé en ce que** ladite plaque d'appui (25) comporte un moyen de raccordement à ladite glissière (2) constitué par un bloc de jonction (32) vissé à ladite plaque pour bloquer la séparation de la glissière (2) également après le montage des autres clamps.
